# DEMANDE DE BREVET EUROPEEN

(11) **EP 1 637 150 A1**
(43) Date de publication de la demande: **22.03.2006**
(21) Numéro de dépôt: 04292187.4
(22) Date de dépôt: 13.09.2004
(51) Int. Cl.: A61K 36/00

(54) **Composition comprenant du Kudzu (Pueraria lobata)**

(71) Demandeur: Condevaux, Georges, 75015 Paris (FR)
(72) Inventeur: Condevaux, Georges, 75015 Paris (FR)
(74) Mandataire: Thinat, Michel

(57) **Abrégé**

La présente invention concerne une composition comprenant Kudzu pour lutter contre la dépendance aux drogues.

L'invention concerne également l'association de Kudzu et de plantes médicinales telles que de la valériane ou de l'aubépine, permettant de compenser de façon ciblée les effets psychologiques liés au manque.

## Description

La présente invention concerne principalement une composition comprenant du Kudzu.

L'invention concerne également l'utilisation du Kudzu pour la fabrication d'une composition servant à lutter contre la dépendance aux drogues.

La désaccoutumance aux drogues nécessite un accompagnement tant thérapeutique que psychologique.

En effet, si les substituts ou équivalents existants actuellement peuvent permettre de compenser le manque physique, l'effet psychologique du manque, et en particulier le stress et/ou l'état dépressif, doit également être pris en considération dans le même temps que le traitement thérapeutique.

Or, la réussite dans la lutte contre la dépendance aux drogues dépend directement de cet équilibre entre l'accoutumance physique et l'accoutumance psychologique à cet effet de manque.

De plus, les substituts ou équivalents peuvent présenter des inconvénients tels que, dans le cas des patchs nicotiniques, des allergies cutanées.

L'invention se situe dans ce contexte en incitant l'utilisateur à diminuer naturellement sa consommation de drogue sans qu'il ressente les effets psychologiques liés au manque et en palliant les inconvénients précités.

A cet effet, l'invention concerne une composition comprenant du Kudzu.

De préférence, la composition de l'invention comprend au moins des racines de Kudzu.

Avantageusement, la composition comprend au moins du kudzu et une plante médicinale.

Dans ce cas, la composition peut comprendre au moins du kudzu et de la valériane pour lutter contre la dépendance au tabac ou au moins du kudzu et de l'aubépine pour lutter contre la dépendance à l'alcool.

Préférentiellement, la composition de l'invention comprend alors environ 300 mg de Kudzu et environ 50 mg d'aubépine ou de valériane.

L'invention concerne également l'utilisation du kudzu pour la fabrication d'une composition servant à lutter contre la dépendance aux drogues.

Dans ce cas, la composition sera avantageusement préparée sous forme de gélule à ingérer.

L'invention sera mieux comprise et d'autres buts, avantages et caractéristiques de celle-ci apparaîtront plus clairement à la lecture de la description qui suit.

Le Kudzu est une plante grimpante d'origine asiatique.

Il a été découvert que l'ingestion de cette plante permettait de lutter efficacement contre la dépendance aux drogues.

Les parties consommables et utilisables de cette plante sont les racines séchés et les fleurs séchées.

On préférera les racines renfermant majoritairement les actifs qui incitent l'utilisateur à consommer moins de drogue.

En effet, on attribue les qualités thérapeutiques du kudzu aux flavonoïdes qu'elle contient tels que la diadzine, la diadzeïne et la puerarine.

Pour lutter contre la dépendance à l'alcool, on choisit d'associer aux racines de Kudzu des fleurs d'aubépine.

Les qualités sédatives et l'action cardiorégulatrice de l'aubépine associées au Kudzu permettent de cibler la dépendance particulière liée à l'alcool.

De cette façon, l'utilisateur se détache plus facilement et sans effets secondaires provenant du manque de la consommation excessive et régulière d'alcool.

Pour lutter contre la consommation de tabac, on associera le kudzu et des racines de valériane.

De la même façon, cette association permet de cibler la dépendance particulière au tabac en apportant par le Kudzu le détachement physique et psychologique à cette drogue sans en ressentir les effets psychologiques du manque de par les qualités sédatives, tranquilisantes et l'activité sur la sphère neuro-psychique de la valériane.

L'association de ces plantes médicinales au kudzu permet, pour l'utilisateur, d'avoir dans le même temps les moyens de se désaccoutumer physiquement et psychologiquement d'une drogue sans ressentir fortement les effets du manque et en particulier le stress et l'effet dépréssif lié à l'arrêt de la prise de drogue.

La lutte contre la dépendance aux drogues en est ainsi nettement améliorée.

Des gélules d'un poids en substance active de 350 mg sont préparés, chaque gélule contenant 300 mg de Kudzu et 50 mg d'Aubépine ou de Valériane selon la drogue ciblée.

La préparation de ces gélules peut se faire par pulvérisation des plantes par cryobroyage permettant avantageusement de sauvegarder les éléments volatils.

Pour obtenir l'effet escompté du kudzu et/ou de l'association du kudzu à d'autres plantes à effet compensatoire, six gélules sont prises dans la journée dont deux le matin, deux le midi et deux le soir. De préférence, la prise de ces gélules s'effectue avec un peu d'eau à l'heure des repas.

Ainsi, une consommation de 1800 mg de kudzu par jour permet de lutter efficacement contre la dépendance aux drogues.

Des résultats satisfaisants de désaccoutumance sont observés très rapidement après le début du traitement.

Les effets de l'ingestion de kudzu ont fait l'objet de tests de non toxicité effectués par le Laboratoire Phycher Bio Développement sur des rats, ces tests ayant abouti à la délivrance d'une attestation d'assurance qualité (étude TAO423-PH-04/0109).

## Revendications

1. Composition comprenant du kudzu et servant à lutter contre la dépendance aux drogues.

2. Composition selon la revendication 1 comprenant au moins des racines de Kudzu.

3. Composition selon l'une quelconque des revendications 1 et 2 comprenant au moins du kudzu et une plante médicinale.

4. Composition selon la revendication 3 comprenant au moins du kudzu et de la valériane pour lutter contre la dépendance au tabac ou au moins du kudzu et de l'aubépine pour lutter contre la dépendance à l'alcool.

5. Composition selon la revendication 4 comprenant environ 300 mg de Kudzu et environ 50 mg d'aubépine ou de valériane.

6. Utilisation du kudzu pour la fabrication d'une composition servant à lutter contre la dépendance aux drogues.

7. Utilisation selon la revendication 6 dans laquelle la composition est préparée sous forme de gélule à ingérer.
